# EUROPÄISCHE PATENTSCHRIFT

(11) **EP 2 186 477 B1**
(45) Veröffentlichungstag und Bekanntmachung des Hinweises auf die Patenterteilung: **13.03.2013**
(21) Anmeldenummer: 09014342.1
(22) Anmeldetag: 17.11.2009
(51) Int. Cl.: A61B 5/103, A61B 19/00

(54) **Nichtinvasiver Sensor zur Untersuchung des Bewegungsapparates von Menschen oder Tieren**
Non-invasive sensor for the examination of the human or animal locomotor system
Capteur non invasif pour l'examen de l'appareil locomoteur humain ou animal

(30) Priorität: 18.11.2008 DE 102008058041
(43) Veröffentlichungstag der Anmeldung: 19.05.2010
(73) Patentinhaber: ITBB Institut für Technologien der Biomechanik und Biomaterialien GmbH, 47506 Neukirchen-Vluyn (DE)
(72) Erfinder: Kecskeméthy, Andrés, 47058 Duisburg (DE)
(74) Vertreter: Schoenen, Norbert

(56) Entgegenhaltungen:
- DE-U1- 20 315 470
- US-A- 6 063 031
- US-A- 6 091 981
- US-A1- 2004 267 165

## Beschreibung

Die Erfindung betrifft eine Einrichtung zur Untersuchung des Bewegungsapparates eines Menschen oder Tieres mit einem Trackingsystem, einem mit dem Trackingsystem verbundenen Rechner und einer Anzeigeeinrichtung zur Wiedergabe des vom Trackingsystem erfassten Erscheinungsbildes, mit Sensoren zur Positionsbestimmung von markanten anatomischen Punkten eines menschlichen oder tierischen Körpers, wobei die Sensoren ein Gehäuse (3) und Marker (4) oder Markerbefestigungen (4a) des Trackingsystems an der Außenseite und ein Flächenelement an der Innenseite aufweisen und wobei das Gehäuse (3) mit seiner Innenseite an dem Körper fixierbar ist, wobei das Gehäuse (3) im Innenteil das Flächenelement (1) zur Bestimmung der Position der markanten anatomischen Punkte aufweist und wobei eine Einrichtung (9, 10) zur Übermittlung der Position an eine Auswerteeinheit (8) vorgesehen ist.

### Nächstliegender Stand der Technik:

Das der Erfindung zugrunde liegende allgemeine Problem ist in DE 203 15 470 U1 dargestellt. Es geht um die Lagebestimmung eines Körperteils mit einem Trackingsystem (Navigationssystem) und Markierelementen, die am Körperteil gehalten sind und deren Lage relativ zum Trackingsystem durch dieses System bestimmt werden soll.

Ebenso wie im Stand der Technik nach DE 203 15 470 U1 soll auch in der Erfindung das Markierelement nicht starr mit festen Strukturen des Körpers, z. B. als Knochenschraube, verbunden sein, sondern sich relativ zu den Knochenstrukturen bewegen können.

Im Stand der Technik nach DE 203 15 470 U1 wird das Problem dadurch gelöst, dass die Markerbefestigungen eines Bewegungstrackingsystems mit einer Lagebestimmungseinrichtung verbunden sind, welche die Lage der festen Strukturen des Körperteils oder eines in den Körper eingebrachten, mit der knöchernen Struktur verbundenen Markierpunktes (engl. 'fiducial') relativ zu den Markern bestimmt und die dieser Lage entsprechenden Lagedaten dem Trackingsystem, also dem Rechner zuführt, welcher aufgrund dieser Lagedaten und der Lagedaten der Marker relativ zum Trackingsystem die Lage der festen Struktur, also der Knochenstrukturen, oder des Markierpunktes, relativ zum Trackingsystem bestimmt. Es handelt sich also um eine doppelte Lagebestimmung einmal der Lage der Marker relativ zur Knochenstruktur und zum anderen der Marker relativ zum Trackingsystem. Für die Lagebestimmung der festen Strukturen des Körperteils relativ zu den Markern muss daher nach DE 203 15 470 U1 die Lagebestimmungseinheit in der Lage sein, die Lage der festen Strukturen des Körperteils relativ zu den Markern des Trackingsystems in allen drei Raumrichtungen zu bestimmen. Nur so kann aus der Erkennung der festen Struktur relativ zu den Markerbefestigungen eines Bewegungstrackingsystems zusammen mit der Information aus dem Navigationssystem die Lage der festen Struktur im festen Bezugssystem bestimmt werden. Dies erfordert Messköpfe mit Sendern und Empfängern, die sowohl in Querrichtung als auch in Tiefenrichtung Gewebestrukturen erkennbar machen können. In der DE 203 15 470 U1 werden als Beispiele für Lagebestimmungseinrichtungen demnach Ultraschallsensoren, Kernspintomographen mit Oberflächenspulen oder Low-Dose X-Ray Digitizers vorgeschlagen.

Vorteilhaft bei solchen räumlichen Lagebestimmungseinrichtungen sind die gute räumliche Auflösung des Innengewebes und die gute Differenzierung von Weichgewebe und knöchernen Strukturen. Dieser guten räumlichen Auflösung stehen jedoch die relativ hohen Gerätekosten einer Ultraschallortung für jeden der Marker und zum anderen das relativ hohe Gewicht von Ultraschallsender und -empfänger, der notwendige Einsatz von Gel zwischen Messkopf und Hautgewebe, damit der Schall nicht durch Lufteinschlüssen zwischen Messkopf und der Hautoberfläche reflektiert wird, sowie die Notwendigkeit des permanenten manuellen Nachjustierens des Messkopfes relativ zum Gewebe während der Messung, nachteilig gegenüber.

Wenn die Lage der Knochen bei einer natürlichen Bewegung des Menschen erfasst werden soll, wie das in der vorliegenden Erfindung geschehen soll, dann behindert das relativ hohe Gewicht und die Sperrigkeit der Ultraschallortung eine natürliche Bewegung des Patienten bzw. Probanden. Aus diesem Grund werden Ultraschallsensoren hauptsächlich für chirurgische Eingriffe im OP oder zur lokalen Untersuchung von Knochenbewegungen unter Laborbedingungen eingesetzt, bei denen der Patient oder die zu untersuchende Person stillsteht, -sitzt oder -liegt. In der Ganganalyse werden diese Sensoren nicht eingesetzt, oder wenn überhaupt, dann nur für sehr restriktive Bewegungsanalysen unter Laborbedingungen, zum Beispiel um die Bewegung des Meniskus bei Kniebeugungen zu untersuchen.

### Weiterer Stand der Technik:

Ein anderer Sensor zur Positionsbestimmung von markanten anatomischen Punkten eines menschlichen Körpers ist aus der DE 103 10 331 B3 bekannt. Es handelt sich hier um einen Ultraschallsensor, nämlich einen Aufnahme- bzw. Schwingerkopf 8 einer Sonographieeinrichtung mit einer Kontaktoberfläche 15.

Aus dieser Entgegenhaltung geht nicht hervor, dass der Sensor 8 oder die Kontaktoberfläche 15 so weich bzw. elastisch oder formbar ist, dass er an die Kontur des Körpers anpassbar ist. Über die Härte und Nachgiebigkeit des Schwingerkopfs 8 und der Kontaktoberfläche 15 wird keine Aussage gemacht. Es ist aber davon auszugehen, dass es sich hier um ein übliches hartes, also formbeständiges Teil handelt.

Ferner geht aus dieser Entgegenhaltung nicht hervor, dass der Sensor als drucksensitives Flächenelement und zur Bestimmung der Druckverteilung ausgebildet ist. Ein drucksensitiver Sensor geht aber aus der DE 43 36 144 A1 hervor. Der Sensor besteht hier aus einer Messwalze 1, die an der Außenfläche rasterförmig angeordnete Druck- oder Wegesensoren aufweist. Die Messwalze wird mit einem Griff 4 manuell auf die zu erfassende Körperoberfläche aufgedrückt. Die von der Messwalze abzuleitenden elektrischen Messsignale können über Schleifkontakte nach außen abgeleitet werden. Bevorzugt ist hier eine Ableitung durch den Griff 4 (Spalte 3, Zeile 62 bis Spalte 4, Zeile 5). Bei einer Ausführungsform der Messwalze mit kapazitiven Sensoren 5 kann zwischen den Messsensoren 5 ein elastisches Dielektrikum 6 angeordnet sein (Spalte 3, Zeilen 52 bis 54).

Ferner geht aus dieser Entgegenhaltung nicht hervor, dass die Knochenlage nicht direkt aus einem Messpunkt - wie bei den genannten Entgegenhaltungen - sondern erfindungsgemäß indirekt aus mehreren simultan gemessenen Druckpunkten über einem komplizierten Algorithmus ermittelt wird. Erst dadurch wird es möglich, den Sensor leicht und kostengünstig zu gestalten, was bei den bestehenden Systemen, die aus einem einzigem Messpunkt direkt die Knochenlage zu ermitteln versuchen, nicht möglich ist und entsprechend zu schweren und sperrigen Hilfsmitteln führt, die für eine Mitführung während der natürlichen Bewegung offensichtlich ungeeignet sind.

Das Verfahren nach der DE 103 10 331 B3 ermöglicht zwar die Bestimmung der Lage der Knochen im Raum bei einer Bewegung des Menschen bzw. Tieres in sehr präziser Weise, ist aber aufgrund der gewählten Messmethode Sonographie relativ kostspielig und die Sensoren sind relativ schwer und sperrig, wodurch eine Messung während der natürlichen Bewegung nicht möglich ist.

Bei der DE 43 36 144 A1 wiederum handelt es sich um ein Messverfahren mit drucksensitiven Sensoren, bei denen der Proband keine Bewegung ausführen kann, ohne die Lokalisierung der knöchernen Merkmale zu verfälschen.

Somit erlauben es die bestehenden Systeme nicht, Knochenbewegungen während einer natürlichen Bewegung präzise zu ermitteln.

Weitere derzeitige Möglichkeiten für die Erfassung von markanten Punkten am Skelett einer Person sind:
- CT
- Röntgen
- ORT
- Marker
- Bonepins
- Untersuchung durch medizinisches Personal (z.B. Arzt /Ärztin)

### Nachteile dieser bestehenden Systeme:

Die Untersuchung des Skelettes einer Zielperson mit CT und Röntgen ist aufgrund der damit verbundenen Strahlenbelastung bedenklich.

Bei CT und MRT können aufgrund des Messverfahren und des eingeschränkten Messraums keine Bewegungen erfasst werden.

Die Markierung von anatomischen Punkten durch Marker ermöglicht keine Verfolgung von anatomischen Punkten mit hinreichender Genauigkeit, weil aufgrund von Hautbewegungen die Messung verfälscht wird.

Die Verwendung von Bonepins zur Markierung von anatomischen Punkten stellt einen medizinischen Eingriff dar, der mit gesundheitlichen Risiken und Schmerzen verbunden ist. Aus diesem Grund ist dieses System für eine breite Anwenderschicht ungeeignet.

Die Untersuchung von anatomischen Punkten durch medizinisches Personal gestattet aufgrund von subjektiven Ergebnissen keine hinreichende Genauigkeit.

Der Erfindung liegt die Aufgabe zugrunde, die oben genannten Nachteile zu vermeiden.

Aufgabe der vorliegenden Erfindung ist außerdem eine Einrichtung zur sehr präzisen Ermittlung der Lage von Knochen im Raum bei der tierischen oder menschlichen Bewegung, aber in erheblich kostengünstigerer Weise mit erheblich leichteren Sensoren, ohne dass die Messungen durch die Hautbewegung verfälscht werden, wodurch ein Einsatz während der natürlichen menschlichen Bewegung möglich ist.

Diese Aufgabe wird bei der Einrichtung zur Untersuchung des Bewegungsapparates der eingangs genannten Art erfindungsgemäß durch die Merkmale des Anspruchs 1 gelöst. Die Kombination aus einer äußeren weitgehend formbeständigen Manschette und einem weichen nachgiebigen Flächenelement im Inneren der Manschette im Raum erlaubt es, sowohl die markanten Punkte an einem Gelenk relativ zur Manschette im Raum zu ermitteln, wodurch die präzise Ermittlung markanter Knochenpunkte ("landmarks") im Raum nichtinvasiv möglich ist.

Erst das Zusammenspiel von einer weichen drucksensitiven Schicht und einem formbeständigen Gehäuse erlaubt es überhaupt, die vorgesehenen Knochenmerkmale während einer natürlichen Bewegung eines Menschen oder Tieres zu messen.

Zur Lösung dieses Problems hat der Erfinder der vorliegenden Anmeldung den beschriebenen Sensor entwickelt, welcher zweiteilig aufgebaut ist und eine harte, formbeständige Außenschale, das "Gehäuse (3)" und einen relativ weichen "Innenmantel (2)" aufweist, welcher an die Kontur des Körpers anpassbar ist. Dadurch ist die Anwendung von drucksensitiven Flächenelementen bei dennoch sehr hoher Genauigkeit der Messungen bei Bewegung des Patienten möglich. Der weiche, das drucksensitive Flächenelement 1 aufweisende Innenmantel 2 liegt eng um den Körper des Patienten und erfasst die Knochenkonturen. Andererseits verhindert das formbeständige Gehäuse 3, an dem die Marker bzw. Markerbefestigungen angebracht sind, eine Beweglichkeit der Marker/Markerbefestigungen relativ zum Gehäuse, wie es bei einem weichen Gehäuse der Fall wäre.

Auf diese Weise findet trotz einer Bewegung des Patienten keine Relativbewegung zwischen dem Marker/Markerbefestigung und den Drucksignalen auf der drucksensitiven Flächenelementen statt, wodurch die Knochen, also die markanten anatomischen Punkte des Körpers präzise erkannt werden können. Der zweiteilige, aus unterschiedlichen Materialien zusammengesetzte Sensor ermöglicht damit aufgrund der einfachen und leichten Bauweise eine hochpräzise Erfassung der markanten anatomischen Punkte während einer natürlichen Bewegung des Patienten.

Ferner unterscheidet sich die hier vorgelegte Patentidee auch deshalb ganz wesentlich von den bestehenden Systemen, weil mit dem hier beschriebenen Sensor die Knochenlage nicht direkt aus einem Messpunkt - wie bei den oben genannten Entgegenhaltungen - sondern indirekt aus mehreren simultan gemessenen Druckpunkten über einen komplizierten Algorithmus ermittelt wird. Erst dadurch wird es möglich, den Sensor leicht und kostengünstig zu gestalten, was bei den bestehenden Systemen, die aus einem einzigem Messpunkt direkt die Knochenlage zu ermitteln versuchen, nicht möglich ist und entsprechend zu schweren und sperrigen Hilfsmitteln führt, die für eine Mitführung während der natürlichen Bewegung offensichtlich ungeeignet sind.

Ferner sei auch darauf hingewiesen, dass eine solche zweiteilige Ausführung des Sensors im Stand der Technik nicht offenbart ist.

Dass die hier vorgestellte Erfindung erfinderisch ist, zeigt sich nicht zuletzt auch daran, dass trotz des sehr großen Praxisbedarfs an präzisen Messungen der Knochenbewegungen während der natürlichen Bewegung eines Menschen oder Tieres, und trotz der sehr verbreiteten Methode der Ganganalyse weltweit, derzeit keine entsprechenden Marktprodukte existieren, obwohl sich dies wie in der vorliegenden Patentanmeldung dargestellt leicht und kostengünstig realisieren lässt.

Erfindungsgemäß wird ein Sensor in Form einer Manschette extern an der Zielperson angebracht. Die Position der Manschette wird durch ein Trackingsystem verfolgt. Aufgrund der Druckverteilung auf einer im Inneren der Manschette angebrachten Messfolie wird die Lage der markanten Punkte mit einer hohen Genauigkeit relativ zur Folie erfasst und danach mit Hilfe der Absolutmessung der Manschette auf einen absoluten Ort im Raum zurückgeführt.

Vorteilhafte Ausgestaltungen der Erfindung sind in den Unteransprüchen dargestellt.

Anstatt eines Gehäuses (3) können auch mehrere formbeständige Außenhüllen verwendet werden, welche jeweils innen mit weichen Flächenelementen verbunden sind und außen Marker oder Markerbefestigungen aufweisen und jeweils auf einem Messpunkt aufgedrückt werden können. Die Verbindung zwischen den Außenhüllen kann über Bänder, Gürtel oder flexible Bügel ähnlich einem Kopfhörer erfolgen.

### Mögliche Anwendungsgebiete:

Bestimmung der genauen Lage von anatomisch markanten Punkten am menschlichen Skelett wie z.B.
- Lage der Kondülen am Kniegelenk,
- Lage der Malleus medialis am Sprunggelenk,
- Lage der Processus styloideus ulnae und Processus styloideus radialis am Handgelenk,
- Lage der Processus styloideus ulnae und Processus styloideus radialis am Ellenbogengelenk,
- Lage der Spina iliaca posterior superior und Spina iliaca anterior superior am Becken,
   zur
- Vorbereitung von chirurgischen Eingriffen in der Orthopädie
- Vorbereitung und Bewertung von Rehabilitationsmaßnahmen in der Orthopädie
- Beurteilung und Optimierung der Skelettbewegung (z.B. von Sportlern)
- Beurteilung von Prozessen auf Ergonomie (Arbeitssicherheit)

Im Folgenden wird ein Ausführungsbeispiel der Erfindung anhand von Zeichnungen näher beschrieben. Es zeigen
- Figur 1: die Explosionsdarstellung eines erfindungsgemäßen Sensors,
- Figur 2: den Sensor nach Figur 1 im montierten Zustand,
- Figur 3: eine erfindungsgemäße Einrichtung zur Unter- suchung des Bewegungsapparates mit dem Sensor nach den Figuren 1 und 2,
- Figur 4: die Druckmessfolie des Sensors nach Figur 1 und
- Figur 5: die grafische Darstellung der Druckverteilung über den Umfang der Druckmessfolie.

In allen Zeichnungen haben gleiche Bezugszeichen die gleiche Bedeutung und werden daher gegebenenfalls nur einmal erläutert.

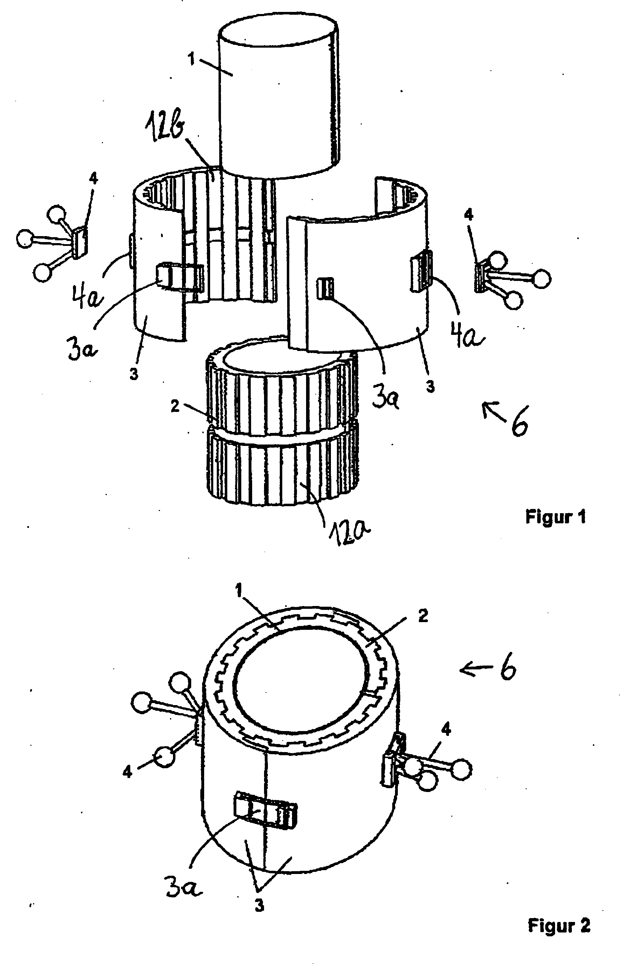

Figur 1 zeigt die Explosionsdarstellung des Sensors (6), während Figur 2 die Zusammenbaudarstellung des Sensors (6) verdeutlicht.

Im Kern des Sensors (6) befindet sich eine Druckmessfolie (1), welche durch eine Klebeverbindung an einer weichen Schicht, z.B. einem Schaumstoffelement (2) fixiert ist. Folie (1) und Schaumstoff (2) können sich somit relativ zueinander nicht verschieben. Um den Schaumstoff (2) wird ein Gehäuse ("Manschette")(3) gelegt, welches weitgehend formbeständig ist bzw. dessen Form sich aus wenigen Messungen ermitteln lässt (z.B. Kunststoffschale). Schaumstoff (2) und Gehäuse (3) sind formschlüssig miteinander verbunden, so dass eine exakte Positionierung des Schaumstoffes (2) relativ zur Manschette (3) gewährleistet ist. Auf dem Gehäuse sind Laschen zur Aufnahme von Markerbäumen (4) befestigt.

Der Sensor (6) dient zur Positionsbestimmung von markanten anatomischen Punkten eines menschlichen oder tierischen Körpers, wobei der Sensor als ein Gehäuse mit bekannter bzw. rechnerisch bestimmbarer Geometrie, zum Beispiel als eine Manschette (2, 3), ausgebildet ist und Marker (4) oder Markerbefestigungen (4a) aufweist und wobei das Gehäuse (2, 3) mit seiner drucksensitiven Innenseite an dem Körper fixierbar ist. Er ist **dadurch gekennzeichnet, dass** der Sensor (2, 3) einerseits ein Element (1) zur Bestimmung der Druckverteilung an der Innenseite des Sensors aufweist und dass eine Einrichtung (9, 10) zur Übermittlung der Druckverteilung an eine Auswerteeinheit (8) vorgesehen ist, und andererseits dass der Sensor über ein äußeres Gehäuse mit bekannter oder rechnerisch bestimmbarer Geometrie verfügt, an dem Marker der Markerbefestigungen angebracht sind, womit die absolute Lage des Gehäuses im Raum ermittelt werden kann. Die Patentidee besteht darin, aus diesen beiden Informationen die absolute Lage markanter Knochenpunkte eines Bewegungsapparates präzise nichtinvasiv experimentell bestimmen zu können.

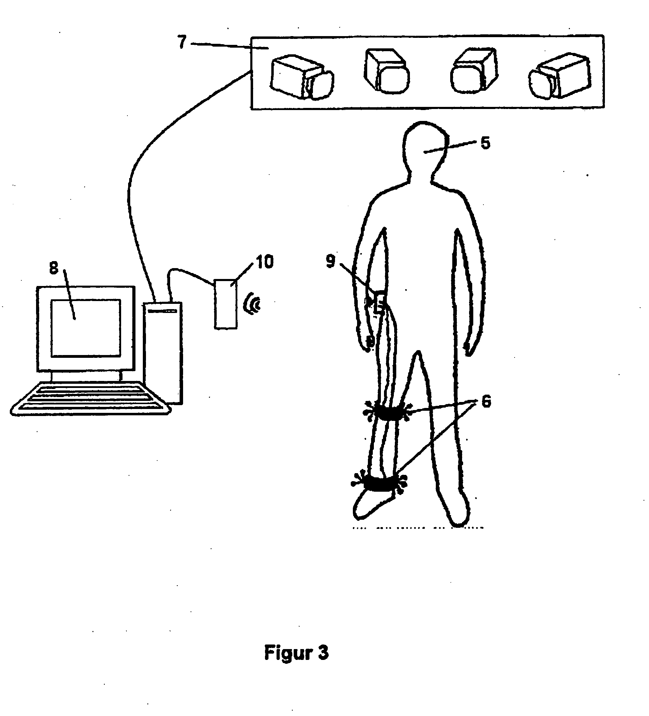

Figur 3 zeigt die Anwendung des Sensors. An der Zielperson (5) werden beliebig viele der erfindungsgemäßen Sensoren (6) an Körperstellen befestigt, an denen markante anatomische Punkte erfasst werden sollen (z.B. an Sprung- und Kniegelenk).

Um die Gehäuse (3) der Anatomie der Zielperson anzupassen, gibt es die Gehäuse (3) in unterschiedlichen Größen. Der dazugehörige Schaumstoff (2) wird in der passenden Dicke gewählt (vgl. Figur 1 und 2). Da der Schaumstoff (2) und die darauf befestigte Messfolie (1) elastisch sind, können sie beliebig aufgebogen und um den zu untersuchenden Körperteil gelegt werden. Der Schaumstoff (2) passt sich daraufhin der Kontur des Körpers an und presst die Folie (1) auf die Haut.

Die Position des Sensors (6) wird durch die darauf befestigten Markerbäume (4) mit Hilfe von Trackingkameras (7) verfolgt und in einem PC (8) ausgewertet.

Die Messergebnisse von dem in dem Sensor (6) befindlichen Druckmessfolie (1) werden über ein Probandenmodem (9) schnurlos an einen Empfänger (10) gesendet und in dem PC (8) mit den Daten der Trackingkameras (7) synchronisiert.

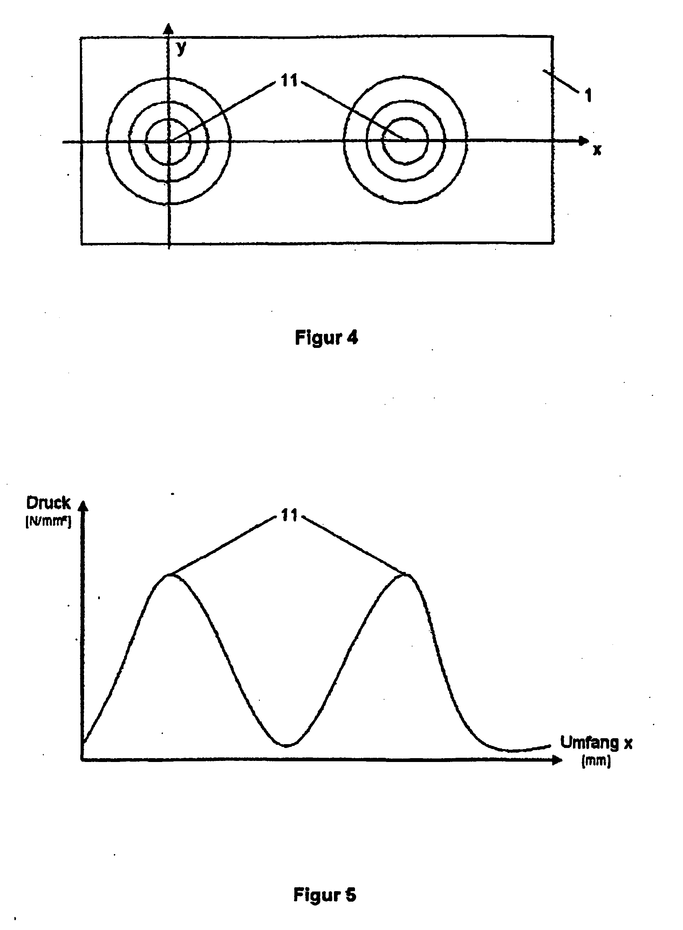

Figur 4 zeigt die qualitative Druckverteilung auf der abgewickelten Druckmessfolie (1). Es ist ersichtlich, dass hervorstehende markante anatomische Punkte auf der Folie ein Druckmaximum (11) hinterlassen. Der qualitative Verlauf des Druckes über dem Umfang ist in Figur 5 dargestellt. Die der Erfindung zu Grunde liegende Idee besteht darin, aus dem Verlauf des Druckes die Maxima (11) zu bestimmen und durch diese Maxima die Position der markanten Punkte auf der Druckmessfolie (1) zu bestimmen. Das gleiche Prinzip lässt sich auch in der transversalen Richtung y anwenden, um so die beiden Koordinaten des Druckmaximums auf der Folie zu bestimmen. Durch Zusammenfassung dieser Ergebnisse mit der vom Trackingsystem bestimmten Position des Sensors im Raum kann nachfolgend die absolute Position der markanten Punkte errechnet werden.

### Bezugszeichenliste

- 1: Druckmessfolie
- 2: Schaumstoff bzw. weiche Schicht
- 3: Gehäuse
- 3a: Befestigungselement
- 4: Markerbaum
- 4a: Markerbefestigung
- 5: Zielperson
- 6: Sensor
- 7: Tracking-Kameras
- 8: PC
- 9: Probandenmodem
- 10: Empfänger
- 11: Maxima des Druckverlaufs
- 12a: Längsprofilierung
- 12b: Längsprofilierung

## Patentansprüche

1. Einrichtung zur Untersuchung des Bewegungsapparates eines Menschen oder Tieres mit einem Trackingsystem, einem mit dem Trackingsystem verbundenen Rechner (8) und einer Anzeigeeinrichtung zur Wiedergabe des vom Trackingsystem erfassten Erscheinungsbildes, mit Sensoren (6) zur Positionsbestimmung von markanten anatomischen Punkten eines menschlichen oder tierischen Körpers, wobei die Sensoren ein Gehäuse (3) und Marker (4) oder Markerbefestigungen (4a) des Trackingsystems an der Außenseite und ein Flächenelement (1) an der Innenseite aufweisen und wobei das Gehäuse (3) mit seiner Innenseite an dem Körper fixierbar ist, wobei das Gehäuse (3) im Innenteil das flächenelement (1) zur Bestimmung der Position der markanten anatomischen Punkte aufweist und wobei eine Einrichtung (9, 10) zur Übermittlung der Position an eine Auswerteeinheit (8) vorgesehen ist, wobei
die Sensoren zweiteilig aufgebaut sind und ein hartes formbeständiges Gehäuse (3) und einen relativ weichen Innenmantel (2) aufweisen, der an die Kontur des Körpers anpassbar ist und eine Druckmessfolie (1) zur Bestimmung der Druckverteilung aufweist,
dass mittels der Sensoren mehrere Druckpunkte eines Knochenverbundes simultan bestimmbar sind, durch einen Algorithmus die präzise Lage des Knochens im Raum ermittelbar ist und die Sensoren leicht und kompakt ausgestaltet sind und so eine Positionsbestimmung während der natürlichen Bewegung erlauben.

2. Einrichtung nach Anspruch 1,
**dadurch gekennzeichnet, dass** der Innenmantel (2) und das Gehäuse (3) auf ihren aneinander anliegenden Flächen eine korrespondierende Profilierung (12a, 12b) aufweisen.

3. Einrichtung nach Anspruch 1,
**dadurch gekennzeichnet, dass** das formbeständige Gehäuse (3) als mehrere formbeständige Außenhüllen ausgebildet ist, welche jeweils innen mit weichen drucksensitiven Flächenelementen verbunden sind und außen Marker oder Markerbefestigungen aufweisen und jeweils auf einem Messpunkt aufgedrückt werden können, wobei die Verbindung zwischen den Außenhüllen über Bänder, Gürtel oder flexible Bügel ähnlich einem Kopfhörer erfolgt.

## Claims

1. Device for examining the locomotor system of a human or animal, comprising a tracking system, a computer (8) connected to the tracking system, and a display device for reproducing the outward appearance captured by the tracking system, comprising sensors (6) for determining the position of prominent anatomical points of a human or animal body, wherein the sensors comprise a housing (3) and markers (4) or marker attachments (4a) of the tracking system on the outer face and a surface element (1) on the inner face and wherein the inner face of the housing (3) can be fixed to the body, wherein the inner part of the housing (3) comprises the surface element (1) for determining the position of the prominent anatomical points and wherein a device (9, 10) for transmitting the position to an evaluation unit (8) is provided, wherein the sensors are constructed in two parts and comprise a hard, dimensionally stable housing (3) and a relatively soft inner shell (2) which can be adapted to the contour of the body and comprises a pressure measuring film (1) for determining the pressure distribution, a plurality of pressure points of a bone joint can be determined simultaneously by means of the sensors, the precise location of the bone in space can be established by means of an algorithm and the sensors are light and compact and thus allow position determination during natural movement.

2. Device according to claim 1, **characterised in that** the inner shell (2) and the housing (3) have corresponding profiling (12a, 12b) on the adjacent faces thereof.

3. Device according to claim 1, **characterised in that** the dimensionally stable housing (3) is designed as a plurality of dimensionally stable outer covers which are each connected on the inside to soft, pressure-sensitive surface elements and on the outside comprise markers or marker attachments and can each be pressed on a measuring point, the outer covers being interconnected via straps, belts or flexible U-shaped members similar to a headset.

## Revendications

1. Dispositif pour l'examen de l'appareil locomoteur d'un être humain ou d'un animal, avec un système de suivi, un calculateur (8) connecté au système de suivi et un dispositif d'affichage pour la reproduction de l'aspect extérieur détecté par le système de suivi, avec des capteurs (6) pour la définition de la position de points anatomiques importants d'un corps humain ou animal, les capteurs présentant un boîtier (3) et des marqueurs (4) ou des fixations de marqueurs (4a) du système de suivi sur le côté extérieur et un élément de surface (1) sur le côté intérieur, et le boîtier (3) pouvant être fixé avec son côté intérieur sur le corps, le boîtier (3) présentant dans la partie intérieure l'élément de surface (1) pour la définition de la position des points anatomiques importants, et un dispositif (9, 10) de transmission de la position à une unité d'analyse (8) étant prévu, les capteurs étant constitués en deux parties et présentant un boîtier (3) dur résistant à la déformation et un manteau intérieur (2) relativement tendre qui peut être adapté au contour du corps et qui présente une feuille de mesure de pression (1) pour la définition de la répartition de pression, plusieurs points de pression d'une liaison osseuse pouvant être définis simultanément au moyen des capteurs, la position précise de l'os dans l'espace pouvant être déterminée par un algorithme, et les capteurs étant réalisés de façon légère et compacte, permettant ainsi une définition de position pendant le mouvement naturel.

2. Dispositif selon la revendication 1, **caractérisé en ce que** le manteau intérieur (2) et le boîtier (3) présentent sur leurs surfaces adjacentes un profil (12a, 12b) correspondant.

3. Dispositif selon la revendication 1, **caractérisé en ce que** le boîtier (3) résistant à la déformation est réalisé en tant que plusieurs enveloppes extérieures résistantes à la déformation qui sont raccordées respectivement intérieurement à des éléments de surface tendres sensibles à la pression et qui présentent extérieurement des marqueurs ou des fixations de marqueurs et qui peuvent être pressées respectivement sur un point de mesure, le raccordement entre les enveloppes extérieures s'effectuant par le biais de bandes, de ceintures ou d'étriers flexibles semblables à un casque d'écoute.
